# EUROPEAN PATENT APPLICATION

(11) **EP 4 261 280 A1**
(43) Date of publication of application: **18.10.2023**
(21) Application number: 21920431.0
(22) Date of filing: 02.03.2021
(51) Int. Cl.: C12N 9/06, C12N 15/53, C12P 13/04

(54) **AMINO ACID DEHYDROGENASE MUTANT AND APPLICATION THEREOF**

(30) Priority: 21.01.2021 CN 202110078514
(71) Applicant: Asymchem Life Science (Tianjin) Co., Ltd., Tianjin 300457 (CN)
(72) Inventor: HONG, Hao, Morrisville North Carolina 27560 (US); JAMES, Gage, Morrisville North Carolina 27560 (US); XIAO, Yi, Tianjin 300457 (CN); ZHANG, Na, Tianjin 300457 (CN); JIAO, Xuecheng, Tianjin 300457 (CN); LI, Rui, Tianjin 300457 (CN); ZHANG, Yanqing, Tianjin 300457 (CN); YANG, Yiming, Tianjin 300457 (CN)
(74) Representative: Müller Schupfner & Partner Patent- und Rechtsanwaltspartnerschaft mbB
(86) International application number: PCT/CN2021/078740
(87) International publication number: WO 2022/156042

(57) **Abstract**

Provided are an amino acid dehydrogenase mutant and use thereof. The amino acid sequence of the amino acid dehydrogenase mutant has a sequence as shown in SEQ ID NO: 1, and sites at which amino acid mutations occur include a K144G site. According to the amino acid dehydrogenase mutant, changes in protein structure and function are achieved, the enzyme activity is improved, and the usage amount of amino acid dehydrogenase is reduced. In addition, the enzyme specificity is also correspondingly improved, and the cost in industrial production of amino acids is reduced.

## Description

### Technical Field

The present disclosure relates to the field of biotechnologies, in particular to an amino acid dehydrogenase mutant and an use thereof.

### Background

Amino acid dehydrogenase (AADH for short), with EC (1.4.1.X) as the enzyme classification number, may catalyze reversible oxidative deamination of amino acids and reductive amination of keto acids, and is an important enzyme in the pathway of amino acid synthesis and metabolism. Herein, the amino acid dehydrogenase has the advantages of high stereoselectivity, high efficiency, mild reaction and environmental friendliness and the like in the synthesis of chiral compounds and the preparation of chiral amines by symmetrical reduction of prochiral keto acids or ketones. In reactions catalyzed by the amino acid dehydrogenase, the participation of cofactors, including reduced nicotinamide adenine dinucleotide (NADH) and oxidized nicotinamide adenine dinucleotide (NAD⁺), is required.

In the process of a ketone reduction reaction, the participation of the reduced cofactor NADH is generally required, and in an actual reaction, the oxidized cofactor NAD⁺ may be added, and then the reduced NADH is regenerated by an appropriate cofactor regeneration system. Common cofactor regeneration systems include glucose and glucose dehydrogenase, formate and formate dehydrogenase, secondary alcohol and secondary alcohol dehydrogenase, phosphite and phosphite dehydrogenase and other similar systems. In general, the replacement of the cofactor regeneration system may not substantially affect the function of the amino acid dehydrogenase.

Although the amino acid dehydrogenase has a wide range of commercial value, its sources are relatively limited, and enzyme-producing strains have a problem of low yield. Therefore, it is of great significance to obtain amino acid dehydrogenase strains with excellent characters by directed evolution means for the production and application of the amino acid dehydrogenase.

### Summary

The present disclosure aims to provide an amino acid dehydrogenase mutant and use thereof, as to solve a technical problem in an existing technology that wild-type amino acid dehydrogenase in the existing technology is not suitable for industrial production.

In order to achieve the above purpose, according to one aspect of the present disclosure, an amino acid dehydrogenase mutant is provided. The amino acid dehydrogenase mutant has an amino acid sequence obtained by the mutation of the amino acid sequence shown in SEQ ID NO: 1, and the mutation comprises a mutation site K144G.

Further, the mutation includes at least one of the following mutation sites: L41I/L/T, G42V, G43N, T115I/L/V, M117L/Y, G118N, T119C/E/L/S, P121M/S/T/W, L135I/V, K184M/N/S, G293Aand G294I/V, herein "/" means "or".

Further, the mutation comprises any one of the following mutation site combinations: K144G+L294I, K144G+L294I+L135V, and K144G+L294I+T115I.

Further, the amino acid dehydrogenase mutant has a K144G+L294I+L135V amino acid mutation, and is co-expressed with formate dehydrogenase FDH.

According to another aspect of the present disclosure, a DNA molecule is provided. The DNA molecule encodes the above amino acid dehydrogenase mutant.

According to another aspect of the present disclosure, a recombinant plasmid is provided. The recombinant plasmid contains the above DNA molecule.

Further, the recombinant plasmid is pET-22a(+), pET-22b(+), pET-3a(+), pET-3d(+), pET-11a(+), pET-12a(+), pET-14b, pET-15b(+), pET-16b(+), pET-17b(+), pET-19b(+), pET-20b(+), pET-21a(+), pET-23a(+), pET-23b(+), pET-24a(+), pET-25b(+), pET-26b(+), pET-27b(+), pET-28a(+), pET-29a(+), pET-30a(+), pET-31b(+), pET-32a(+), pET-35b(+), pET-38b(+), pET-39b(+), pET-40b(+), pET-41a(+), pET-41b(+), pET-42a(+), pET-43a(+), pET-43b(+), pET-44a(+), pET-49b(+), pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-8, pUC-18, pRSFDuet1 or pUC-19.

According to another aspect of the present disclosure, a host cell is provided. The host cell contains any one of the above recombinant plasmids.

Further, the host cell includes a prokaryotic cell or a eukaryotic cell; and preferably, the prokaryotic cell is *Escherichia coli* BL21 cell or *Escherichia coli* DH5α competent cell, and the eukaryotic cell is yeast.

According to another aspect of the present disclosure, a method for producing an amino acid is provided. The method includes a step of catalyzing reductive amination of a ketone compound by amino acid dehydrogenase, and the amino acid dehydrogenase is any one of the above amino acid dehydrogenase mutants.

Further, the ketone compound is 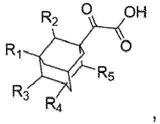 and a reductive amination reaction product is 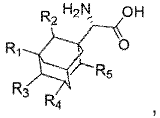 herein R₁ represents -OH, -F, -Cl, -Br or -CH₃, and R₂, R₃, R₄ and R₅ each independently represent -H, -OH, -F, -Cl, -Br or -CH₃; and preferably, the ketone compound is

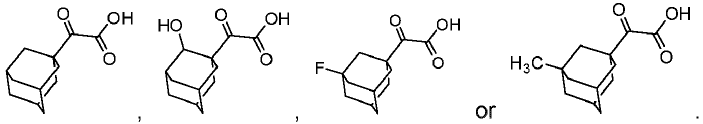

Further, an amino donor in the catalytic reduction ammoniation reaction is ammonium formate, ammonium chloride, ammonium carbamate, ammonium carbonate, ammonium bicarbonate, aqueous ammonia, ammonium oxalate, ammonium hydrogen oxalate, ammonium lactate or ammonium phosphate; and preferably, the reductive amination catalyzing by the amino acid dehydrogenase has a condition of 30°C to 50°C, a stirring speed of 50 rpm to 250 rpm, and a concentration of formate dehydrogenase of 0.1 mg/mL to 1 mg/mL.

The above amino acid dehydrogenase mutant of the present disclosure is based on the amino acid dehydrogenase shown in SEQ ID NO: 1, and is mutated by a method of site-directed mutation, as to change its amino acid sequence and achieve changes of the protein structure and function, and then the amino acid dehydrogenase with the above mutation site is obtained by a method of directional screening. The amino acid dehydrogenase mutant of the present disclosure has the advantage of greatly improving the enzyme activity, thereby the usage amount of the amino acid dehydrogenase is reduced, and the enzyme specificity is also correspondingly improved, thus the cost in industrial production of amino acids is greatly reduced.

### Detailed Description of the Embodiments

It should be noted that embodiments in the present application and features in the embodiments may be combined with each other without conflicting. The present disclosure is described in detail below in combination with the embodiments.

In a patent (CN108795893A), an amino acid dehydrogenase mutantA135L (Template) derived from *Thermoactinomyces intermedius* ATCC33205 may catalyze a target substrate to obtain a product, but the catalytic activity is relatively poor. The present disclosure improves its catalytic activity and reduces the usage amount of amino acid dehydrogenase by a method of directional evolution. The template amino acid sequence of the present disclosure is SEQ ID NO: 1 (MRDVFEMMDRYGHEQVIFCRHPQTGLKAIIALHNTTAGPALGGCRMIPYASTDEALEDVLRLSKG MTYKCSLADVDFGGGKMVIIGDPKKDKSPELFRVIGRFVGGLNGRFYTGTDMGTNPEDFVHAARE SKSFLGLPKSYGGKGDTSIPTALGVFHGMRATARFLWGTDQLKGRVVAIQGVGKVGERLLQLLVE VGAYCKIADIDSVRCEQLKEKYGDKVQLVDVNRIHKESCDIFSPCAKGGVVNDDTIDEFRCLAIVGS ANNQLVEDRHGALLQKRSICYAPDYLVNAGGLIQVADELEGFHEERVLAKTEAIYDMVLDIFHRAK NENITTCEAADRIVMERLKKLTDIRRILLEDPRNSARRLE *,* represents an amino acid termination codon), the corresponding nucleotide sequence is SEQ ID NO: 2 (ATGCGTGACGTATTCGAAATGATGGATCGCTACGGCCACGAGCAGGTGATTTTCTGTCGTCA TCCGCAGACTGGCCTGAAAGCGATCATCGCTCTGCATAACACCACTGCCGGTCCGGCACTGG GCGGTTGTCGCATGATTCCATACGCAAGCACCGATGAAGCTCTGGAAGACGTTCTGCGTCTG AGCAAAGGTATGACCTATAAATGCTCTCTGGCGGATGTTGATTTCGGTGGCGGTAAAATGGTG ATTATCGGCGATCCGAAAAAGGATAAAAGCCCAGAACTGTTCCGTGTTATCGGTCGCTTCGTT GGCGGCCTGAACGGTCGTTTCTATACCGGTACTGATATGGGCACCAATCCGGAAGATTTCGT GCACGCCGCTCGCGAAAGCAAATCTTTTCTAGGTCTGCCTAAATCTTACGGTGGTAAAGGTGA CACTTCTATCCCGACCGCACTGGGTGTATTTCACGGCATGCGCGCGACCGCCCGCTTTCTGT GGGGCACCGATCAACTGAAAGGTCGTGTTGTTGCTATCCAGGGTGTTGGCAAAGTGGGTGAA CGTCTGCTGCAGCTGCTGGTGGAAGTGGGTGCATACTGCAAAATTGCTGATATTGACTCTGTA CGTTGTGAGCAGCTGAAAGAAAAGTACGGCGACAAAGTCCAGCTGGTAGACGTGAACCGTAT CCACAAAGAGTCTTGTGACATCTTCTCCCCGTGCGCAAAAGGCGGCGTAGTCAACGACGACA CTATTGACGAATTCCGCTGCCTGGCGATTGTTGGTTCCGCGAACAATCAGCTGGTTGAAGATC GTCATGGCGCGCTGCTGCAAAAACGCTCCATTTGCTATGCCCCGGATTATCTGGTTAACGCT GGCGGTCTGATCCAGGTCGCAGACGAACTGGAGGGTTTTCACGAGGAGCGTGTGCTGGCGA AAACGGAAGCCATCTACGACATGGTTCTGGACATCTTCCACCGCGCTAAGAACGAAAACATCA CTACCTGCGAAGCAGCGGACCGTATCGTAATGGAACGTCTGAAGAAGCTGACGGACATCCGT CGTATCCTGCTGGAAGATCCGCGTAACTCCGCGCGTCGTCTCGAGTGA)

Firstly, a mutation site is introduced into the amino acid dehydrogenase by a mode of site-directed mutation, and the activity of the mutant is detected to select the mutant with improved activity. Compared with an initial template, the activity of the mutant K144G is increased by about 5 times. Subsequently, the mutant K144G is used as a template to continue the mutation in order to obtain a mutant with more significant improved catalytic activity.

Herein, site-directed mutation: referring to introduction of required changes (usually changes that represent favorable directions) into a target DNA segment (either a genome or a plasmid) by a polymerase chain reaction (PCR) or other methods, including base addition, deletion, point mutation and the like. The site-directed mutation may rapidly and efficiently improve the character and representation of a target protein expressed by DNA, and is a very useful means in gene research work.

A method of introducing the site-directed mutation by the whole-plasmid PCR is simple and effective, and is more widely used at present. The principle thereof is that a pair of primers (forward and reverse) containing mutation sites are annealed with a template plasmid, "cyclic extension" is performed by using a polymerase, and the so-called cyclic extension is that the polymerase extends the primer according to the template, is returned to a 5'-terminal of the primer and terminated after one circle, and subjected to a cycle of repeatedly heated and annealed extension, this reaction is different from rolling circle amplification, and does not form multiple tandem copies. Extension products of the forward and reverse primers are paired to form an open-circle plasmid with an incision after annealed. A Dpn I enzyme-digested extension product, because the original template plasmid is derived from conventional Escherichia coli, is modified by dam methylation, and is sensitive to Dpn I so as to be shredded, but a plasmid with a mutant sequence synthesized in vitro is not cut because it is not methylated, so it may be successfully transformed in subsequent transformation, and a clone of a mutant plasmid may be obtained. The mutant plasmid is transformed into *Escherichia coli* cells, and then a crude enzyme is obtained by ultrasonic cell-break.

The mutant plasmid described above is transformed into the *Escherichia coli* cells and overexpressed in the *Escherichia coli.* Then the crude enzyme is obtained by the method of ultrasonic cell-break. The optimum condition for induced expression of the e amino acid dehydrogenase is: 25°C, and inducing in 0.1 mM isopropyl-β-d-thiogalactoside (IPTG) for 16 h.

By computer simulation analysis of the three-dimensional structure of the amino acid dehydrogenase by software, it is found that most of the mutation sites are located near the active center, and the combination of substrate and enzyme may be enhanced after the mutation, thereby the catalytic efficiency is improved. According to a typical implementation of the present disclosure, an amino acid dehydrogenase mutant is provided. The amino acid dehydrogenase mutant has an amino acid sequence obtained by the mutation of the amino acid sequence shown in SEQ ID NO: 1, and the mutation comprises a mutation site K144G.

Preferably, the mutation includes at least one of the following mutation sites: L41I/L/T, G42V, G43N, T115I/L/V, M117L/Y, G118N, T119C/E/L/S, P121M/S/T/W, L135I/V, K184M/N/S, G293A and G294I/V, herein "/" means "or"; and more preferably, the mutation comprises any one of the following mutation site combinations: K144G+L294I, K144G+L294I+L135V, and K144G+L294I+T115I.

According to a typical implementation of the present disclosure, the amino acid dehydrogenase mutant has a K144G+L294I+L135V amino acid mutation, and is co-expressed with formate dehydrogenase FDH.

The above amino acid dehydrogenase mutant of the present disclosure is based on the amino acid dehydrogenase shown in SEQ ID NO: 1, and is mutated by a method of site-directed mutation, as to change its amino acid sequence and achieve changes of the protein structure and function, and then the amino acid dehydrogenase with the above mutation site is obtained by a method of directional screening. The amino acid dehydrogenase mutant of the present disclosure has the advantage of greatly improving the enzyme activity, thereby the usage amount of the amino acid dehydrogenase is reduced, and the enzyme specificity is also correspondingly improved, thus the cost in industrial production is greatly reduced.

According to a typical implementation of the present disclosure, a DNA molecule is provided. The amino acid dehydrogenase encoded by the above DNA may improve the enzyme activity and stability, the amount of the enzyme added in industrial production of amino acids may be reduced, and the difficulty of post-treatment is reduced.

The above DNA molecule of the disclosure may also exist in the form of an "expression cassette". The "expression cassette" refers to a linear or circular nucleic acid molecule that encompasses DNA and RNA sequences capable of guiding expression of a specific nucleotide sequence in an appropriate host cell. Generally, including a promoter which is effectively linked with a target nucleotide, it is optionally effectively linked with a termination signal and/or other control elements. The expression cassette may also include a sequence required for proper translation of the nucleotide sequence. A coding region usually encodes a target protein, but also encodes a target function RNA in a sense or antisense direction, for example an antisense RNA or an untranslated RNA. The expression cassette including a target polynucleotide sequence may be chimeric, which means that at least one of components thereof is heterologous to at least one of the other components thereof. The expression cassette may also be existent naturally, but obtained with effective recombinant formation for heterologous expression.

According to a typical embodiment of the present disclosure, a recombinant plasmid is provided. The recombinant plasmid contains any one of the above DNA molecules. The DNA molecule in the above recombinant plasmid is placed in an appropriate position of the recombinant plasmid, so that the above DNA molecule may be correctly and smoothly replicated, transcribed or expressed.

Although a qualifier used in the disclosure while the above DNA molecule is defined is "contain", it does not mean that other sequences which are not related to a function thereof may be arbitrarily added to both ends of the DNA sequence. Those skilled in the art know that in order to meet the requirements of recombination operations, it is necessary to add suitable enzyme digestion sites of a restriction enzyme at two ends of the DNA sequence, or additionally increase a start codon, a termination codon and the like, therefore, if the closed expression is used for defining, these situations may not be covered truly.

A term "plasmid" used in the present disclosure includes any plasmid, cosmid, bacteriophage or agrobacterium binary nucleic acid molecule in double-stranded or single-stranded linear or circular form, preferably a recombinant expression plasmid, which may be a prokaryotic expression plasmid or may be a eukaryotic expression plasmid, preferably the prokaryotic expression plasmid, in some embodiments, the recombinant plasmid is selected from pET-22a (+) , pET-22b (+) , pET-3a (+) , pET-3d (+) , pET-11a (+) , pET-12a (+) , pET-14b (+) , pET-15b (+) , pET-16b (+) , pET-17b (+) , pET-19b (+) , pET-20b (+) , pET-21a (+) , pET-23a (+) , pET-23b (+) , pET-24a (+) , pET-25b (+) , pET-26b (+) , pET-27b (+) , pET-28a (+) , pET-29a (+) , pET-30a (+) , pET-31b (+) , pET-32a (+) , pET-35b (+) , pET-38b (+) , pET-39b (+) , pET-40b (+) , pET-41a (+) , pET-41b (+) , pET-42a (+) , pET-43a (+) , pET-43b (+) , pET-44a (+) , pET-49b (+) , pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-8, pUC-18, pRSFDuet1, or pUC-19. More preferably, the above recombinant plasmid is pET-22b (+).

According to a typical implementation of the present disclosure, a host cell is provided. The host cell contains any one of the above recombinant plasmids. The host cell applicable to the present disclosure includes but is not limited to a prokaryotic cell or a eukaryotic cell. Preferably the prokaryotic cell is *Escherichia coli* BL21 cell or *Escherichia coli* DH5α competent cell, and the eukaryotic cell is yeast.

According to a typical implementation of the present disclosure, a method for producing an amino acid is provided. The method includes a step of catalyzing reductive amination of a ketone compound by amino acid dehydrogenase, and the amino acid dehydrogenase is any one of the above amino acid dehydrogenase mutants. Because the above amino acid dehydrogenase mutant of the present disclosure has the higher enzyme catalytic activity, the amino acid prepared by the amino acid dehydrogenase mutant of the present disclosure may not only reduce the production cost, but also obtain a higher excessive enantiomer (e.e.) value of the amino acid.

According to a typical implementation of the present disclosure, the ketone compound is 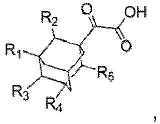 and a reductive amination reaction product is 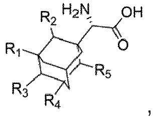 herein R₁ represents -OH, -F, -Cl, -Br or -CH₃, and R₂, R₃, R₄ and R₅ each independently represent -H, -OH, -F, -Cl, -Br or -CH₃; and preferably, the ketone compound is 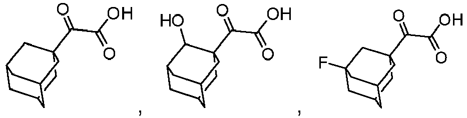 or

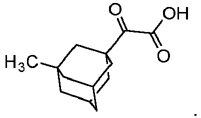

Preferably, the amino donor in the catalytic reduction ammoniation reaction is ammonium formate, ammonium chloride, ammonium carbamate, ammonium carbonate, ammonium bicarbonate, aqueous ammonia, ammonium oxalate, ammonium hydrogen oxalate, ammonium lactate or ammonium phosphate; and the reductive amination catalyzing by the amino acid dehydrogenase has a condition of 30°C to 50°C, a stirring speed of 50 rpm to 250 rpm, and a concentration of formate dehydrogenase of 0.1 mg/mL to 1 mg/mL.

The beneficial effects of the present disclosure are further described below in combination with specific embodiments.

Reaction validation of Substrate 1, 2, 3 and 4:
Substrate 1: 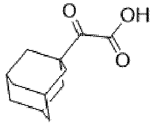 2-(1-adamantane)-2-oxoacetic acid
Substrate 2: 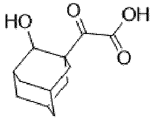 2-(2-hydroxy-1-adamantane)-2-oxoacetic acid
Substrate 3: 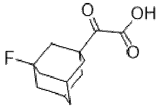 2-(3-fluoro-1-adamantane)-2-oxoacetic acid
Substrate 4: 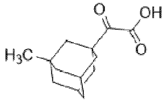 2-(3-methyl-1-adamantane)-2-oxoacetic acid

### Embodiment 1

Mutant A135L (Template, herein A135L represented that an amino acid A at the 135-th site is replaced by L) was subjected to a site-directed mutation. There were 40 mutants at 6 mutation sites including T115, L135, K144, T147, L294 and V297, reaction validation was performed on Substrate 1 and Substrate 2, and a reaction system was: 0.1 g of the substrate, 0.001 g of amino acid dehydrogenase, 0.001 g of formate dehydrogenase, 0.0256 mg of NAD⁺, 0.056 g of ammonium formate, 0.1 M of potassium phosphate solution pH 8.0, 1.1 mL, 40°C, and 16 h, herein the transformation rate of the K144G mutant was the highest. Specific results were shown in Table 1.

**Table 1**

| Mutant | Substrate 1 activity | Substrate 1 e.e.% | Substrate 2 activity | Substrate 2 e.e.% |
|---|---|---|---|---|
| Template | - | >99 | - | >99 |
| M66G | - | >99 | - | >99 |
| T115C | + | >99 | + | >99 |
| T115G | - | >99 | + | >99 |
| T115I | +++ | >99 | +++ | >99 |
| T115L | + | >99 | + | >99 |
| T115M | + | >99 | ++ | >99 |
| T115Q | +++ | >99 | ++ | >99 |
| T115V | ++ | >99 | ++ | >99 |
| L135D | - | >99 | - | >99 |
| L135K | ++ | >99 | + | >99 |
| L135Q | - | >99 | - | >99 |
| L135S | ++ | >99 | + | >99 |
| L135T | ++ | >99 | +++ | >99 |
| L135V | ++ | >99 | ++ | >99 |
| K144C | +++ | >99 | ++ | >99 |
| K144E | ++ | >99 | + | >99 |
| K144G | ++++ | >99 | ++++ | >99 |
| K144H | ++ | >99 | ++ | >99 |
| K144L | +++ | >99 | ++ | >99 |
| K144P | +++ | >99 | +++ | >99 |
| K144S | +++ | >99 | ++ | >99 |
| K144Y | ++ | >99 | + | >99 |
| T147K | - | >99 | - | >99 |
| T147M | - | >99 | - | >99 |
| T147S | + | >99 | + | >99 |
| T147V | - | >99 | - | >99 |
| T147Y | - | >99 | - | >99 |
| L294A | - | >99 | - | >99 |
| L294C | ++ | >99 | ++ | >99 |
| L294I | ++ | >99 | +++ | >99 |
| L294L | + | >99 | + | >99 |
| L294M | ++ | >99 | ++ | >99 |
| L294S | ++ | >99 | ++ | >99 |
| L294V | ++ | >99 | ++ | >99 |
| V297A | + | >99 | - | >99 |
| V297D | - | >99 | - | >99 |
| V297G | - | >99 | - | >99 |
| V297K | - | >99 | - | >99 |
| V297M | - | >99 | - | >99 |
| V297V | ++ | >99 | ++ | >99 |

| | | | | |
|---|---|---|---|---|
| Note: - represents 0.5-20%, + represents 20-40%, ++ represents 40-60%, +++ represents 60-80%, ++++ represents 80-90%, and +++++ represents more than 90%. | | | | |

### Embodiment 2

Mutant K144G was used as a template, and the mutation was continued to perform at 13 sites of L41, G42, G43, G114, T115, M117, G118, T119, P121, L135, K184, G293 and L294, to obtain a total of 43 mutants. Reaction validation was performed on Substrate 1 and Substrate 2, and the reaction conditions were: 0.1 g of the substrate, 0.0005 g of amino acid dehydrogenase, 0.001 g of formate dehydrogenase, 0.0256 mg of NAD⁺, 0.056 g of ammonium formate, 0.1 M of potassium phosphate solution pH 8.0, 1 mL, 40°C, and 16 h, herein after L135V, T115I and L294I sites were added, the transformation rate was improved. Specific results were shown in Table 2.

**Table 2**

| Mutant | Substrate 1 activity | Substrate 1 e.e.% | Substrate 2 activity | Substrate 2 e.e.% |
|---|---|---|---|---|
| K144G | +++ | >99 | +++ | >99 |
| K144G+L41I | +++ | >99 | +++ | >99 |
| K144G+L41K | - | >99 | - | >99 |
| K144G+L41L | +++ | >99 | +++ | >99 |
| K144G+L41S | - | >99 | - | >99 |
| K144G+L41T | +++ | >99 | +++ | >99 |
| K144G+G42V | +++ | >99 | +++ | >99 |
| K144G+G43N | +++ | >99 | +++ | >99 |
| K144G+G114S | - | >99 | - | >99 |
| K144G+T115I | ++++ | >99 | ++++ | >99 |
| K144G+T115L | +++ | >99 | +++ | >99 |
| K144G+T115V | +++ | >99 | +++ | >99 |
| K144G+M117A | - | >99 | - | >99 |
| K144G+M117L | +++ | >99 | - | >99 |
| K144G+M117T | - | >99 | - | >99 |
| K144G+M117V | +++ | >99 | +++ | >99 |
| K144G+M117Y | +++ | >99 | +++ | >99 |
| K144G+G118A | +++ | >99 | +++ | >99 |
| K144G+G118H | - | >99 | - | >99 |
| K144G+G118N | + | >99 | - | >99 |
| K144G+G118Q | - | >99 | - | >99 |
| K144G+G118S | - | >99 | - | >99 |
| K144G+G118Y | - | >99 | - | >99 |
| K144G+T1 19A | - | >99 | - | >99 |
| K144G+T119C | +++ | >99 | +++ | >99 |
| K144G+T119E | +++ | >99 | +++ | >99 |
| K144G+T119L | +++ | >99 | +++ | >99 |
| K144G+T119N | - | >99 | - | >99 |
| K144G+T119S | +++ | >99 | +++ | >99 |
| K144G+P121M | +++ | >99 | +++ | >99 |
| K144G+P121S | +++ | >99 | +++ | >99 |
| K144G+P121T | +++ | >99 | +++ | >99 |
| K144G+P121W | +++ | >99 | +++ | >99 |
| K144G+L135I | ++++ | >99 | ++++ | >99 |
| K144G+L135V | ++++ | >99 | +++ | >99 |
| K144G+K184H | +++ | >99 | - | >99 |
| K144G+K184M | +++ | >99 | - | >99 |
| K144G+K184N | +++ | >99 | +++ | >99 |
| K144G+K184S | +++ | >99 | +++ | >99 |
| K144G+G293A | +++ | >99 | +++ | >99 |
| K144G+G293P | - | >99 | - | >99 |
| K144G+G293S | - | >99 | - | >99 |
| K144G+L294I | ++++ | >99 | ++++ | >99 |
| K144G+L294V | +++ | >99 | +++ | >99 |

| | | | | |
|---|---|---|---|---|
| Note: - represents 0.5-20%, + represents 20-40%, ++ represents 40-60%, +++ represents 60-80%, ++++ represents 80-90%, and +++++ represents more than 90%. | | | | |

### Embodiment 3

A combined saturation mutation might obtain a mutant with a synergistic effect among several mutation sites, and might optimize compositions of its amino acid. Mutant K144G+L294I was used as a template, and mutation points L135V and T115I with the better reaction were superimposed respectively. The reaction conditions were: 0.1 g of the substrate, 0.0005 g of amino acid dehydrogenase, 0.001 g of formate dehydrogenase, 0.0256 mg of NAD⁺, 0.056 g of ammonium formate, 0.1 M of potassium phosphate solution pH 8.0, 1 mL, 40°C, 100 rpm, and 16 h, herein the highest activity was K144G+L294I+L135V. Results were shown in Table 3.

**Table 3**

| Mutation site | Substrate 1 activity | Substrate 1 e.e.% | Substrate 2 activity | Substrate 2 e.e.% |
|---|---|---|---|---|
| K144G | +++ | >99 | +++ | >99 |
| K144G+L294I | ++++ | >99 | ++++ | >99 |
| K144G+L294I+L135V | +++++ | >99 | +++++ | >99 |
| K144G+L294I+T115I | ++++ | >99 | ++++ | >99 |

| | | | | |
|---|---|---|---|---|
| Note: - represents 0.5-20%, + represents 20-40%, ++ represents 40-60%, +++ represents 60-80%, ++++ represents 80-90%, and +++++ represents more than 90%. | | | | |

### Embodiment 4

Reaction scale-up of Substrates 1, 2, 3 and 4: K144G+L294I+L135V was subjected to a reaction and e.e. was detected, and the reaction conditions were: 1 g of the substrate, 0.005 g of amino acid dehydrogenase, 0.01 g of formate dehydrogenase, 0.256 mg of NAD⁺, 0.056 g of ammonium formate, 0.1 M of potassium phosphate solution pH 8.0, 1 mL, 40°C, and 100 rpm.

Sampling method for activity detection: 0.5 mL of a reaction system was taken out, it was centrifuged at 12000 rpm for 5min, 0.050 mL of a supernatant was taken and 1 mL of purified water was added. After being mixed uniformly, it was sent to high performance liquid chromatography (HPLC) for detection of transformation rate.

Sampling method for e.e detection: 1 mL of a reaction transformation system was taken, and pH was adjusted to 10-11 by using 1 M of NaOH; 0.1 mL of (BOC)₂O was added at 30°C and 200 rpm for 0.5 h, and pH was adjusted to 10-11 by using 1 M of NaOH at 30°C and 200 rpm for 0.5 h; the equal volume of isopropyl acetate was added, pH was adjusted to 2.0 by using 6 M of HCl, 0.5 mL of the system was taken and 1 mL of ethyl acetate was added to shake and mix uniformly; and it was centrifuged at 12000 rpm for 5 min, a supernatant was taken and blown dry, anhydrous ethanol was added to dissolve and it was sent to e.e detection. Results were shown in Table 4.

**Table 4**

| Mutant | Substrate 1 | Substrate 2 | Substrate 3 | Substrate 4 | | | | |
|---|---|---|---|---|---|---|---|---|
| | Activity | e.e.% | Activity | e.e.% | Activity | e.e.% | Activity | e.e.% |
| Template | - | >99 | - | >99 | - | >99 | - | >99 |
| K144G | +++ | >99 | +++ | >99 | +++ | >99 | +++ | >99 |
| K144G+L294I+L135V | +++++ | >99 | +++++ | >99 | +++++ | >99 | +++++ | >99 |

| | | | | | | | | |
|---|---|---|---|---|---|---|---|---|
| Note: - represents 0.5-20%, + represents 20-40%, ++ represents 40-60%, +++ represents 60-80%, ++++ represents 80-90%, and +++++ represents more than 90%. | | | | | | | | |

### Embodiment 5

A K144G+L294I+L135V mutant was selected to optimize formate dehydrogenase, and the reaction conditions were: 0.1 g of the substrate, 0.0005 g of amino acid dehydrogenase, 0.0256 mg of NAD⁺, 0.056 g of ammonium formate, 0.1 M of potassium phosphate solution pH 8.0, 1 mL, 40°C, and 100 rpm, herein the activity of the formate dehydrogenase was significantly reduced only while it was less than 0.3 mg, and it was indicated that the formate dehydrogenase was a non-restrictive factor in the reaction process. Results were shown in Table 5.

**Table 5**

| Formate dehydrogenase (mg) | Substrate 1 activity |
|---|---|
| 0.05 | ++ |
| 0.1 | +++ |
| 0.2 | ++++ |
| 0.3 | +++++ |
| 0.4 | +++++ |
| 0.5 | +++++ |
| 0.6 | +++++ |
| 0.7 | +++++ |
| 0.8 | +++++ |
| 0.9 | +++++ |
| 1 | +++++ |

| | |
|---|---|
| Note: - represents 0.5-20%, + represents 20-40%, ++ represents 40-60%, +++ represents 60-80%, ++++ represents 80-90%, and +++++ represents more than 90%. | |

### Embodiment 6

Under the condition of 0.3 mg of formate dehydrogenase, the enzyme amount of K144G+L294I+L135V was optimized, and the reaction conditions were: 0.1 g of the substrate, 0.0256 mg of NAD⁺, 0.056 g of ammonium formate, 0.1 M of potassium phosphate solution pH 8.0, 1.1 mL, 40°C, and 100 rpm. With the decrease of the enzyme amount, the transformation rate was decreased significantly, it was indicated that amino acid dehydrogenase was a restrictive factor in the reaction. Results were shown in Table 6.

**Table 6**

| Amino acid dehydrogenase (mg) | Substrate 1 activity |
|---|---|
| 1 | + |
| 2 | ++ |
| 3 | +++ |
| 4 | ++++ |
| 5 | +++++ |

| | |
|---|---|
| Note: - represents 0.5-20%, + represents 20-40%, ++ represents 40-60%, +++ represents 60-80%, ++++ represents 80-90%, and +++++ represents more than 90%. | |

### Embodiment 7

The reaction conditions of co-expression strains were optimized, K144G+L294I+L135V and formate dehydrogenase FDH were constructed into a co-expression plasmid pRSFDuet1, and transformed into *E.coli* BL21 (DE3). Reaction validation is performed on Substrate 1, and the reaction conditions, such as the temperature and the rotation speed, were optimized, and the reaction conditions were: 0.1 g of the substrate, 0.005 g of amino acid dehydrogenase, 0.0256 mg of NAD⁺, 0.1 M of potassium phosphate solution pH 8.0, and 1.1 mL. Results showed that the optimum reaction conditions were 40°C and 100 rpm. The results were shown in Table 7.

**Table 7**

| Mutant | Temperature (°C) | Rotation speed (rpm) | NAD⁻ (mg) | Substrate 1 activity |
|---|---|---|---|---|
| K144G+L294I+L135V+FDH | 30 | 100 | 0.025 | +++ |
| | 40 | | | +++++ |
| | 50 | | | + |
| | 30 | 200 | | +++ |
| | 40 | | | ++++ |
| | 50 | | | ++ |

| | | | | |
|---|---|---|---|---|
| Note: - represents 0.5-20%, + represents 20-40%, ++ represents 40-60%, +++ represents 60-80%, ++++ represents 80-90%, and +++++ represents more than 90%. | | | | |

### Embodiment 8

Reaction scale-up of Substrates 1, 2, 3 and 4: co-expression strain K144G+L294I+L135V+FDH was subjected to a reaction and e.e. was detected, and the reaction conditions were: 1 g of the substrate, 0.05 g of amino acid dehydrogenase, 0.256 mg of NAD⁺, 0.1 M of potassium phosphate solution pH 8.0, 1 mL, 40°C, and 100 rpm.

Sampling method for activity detection: 0.5 mL of a reaction system was taken out, it was centrifuged at 12000 rpm for 5 min, 0.050 mL of a supernatant was taken and 1 mL of purified water was added. After being mixed uniformly, it was sent to HPLC for detection of transformation rate.

Sampling method for e.e detection: 1 mL of a reaction transformation system was taken, and pH was adjusted to 10-11 by using 1 M of NaOH; 0.1 mL of (BOC)₂O was added at 30°C and 200 rpm for 0.5 h, and pH was adjusted to 10-11 by using 1 M of NaOH at 30°C and 200 rpm for 0.5 h; the equal volume of isopropyl acetate was added, pH was adjusted to 2.0 by using 6 M of HCl, 0.5 mL of the system was taken and 1 mL of ethyl acetate was added to shake and mix uniformly; and it was centrifuged at 12000 rpm for 5 min, a supernatant was taken and blown dry, anhydrous ethanol was added to dissolve and it was sent to e.e detection. Results were shown in Table 8.

**Table 8**

| Mutant | Substrate 1 | Substrate 2 | Substrate 3 | Substrate 4 | | | | |
|---|---|---|---|---|---|---|---|---|
| | Activity | e.e.% | Activity | e.e.% | Activity | e.e.% | Activity | e.e.% |
| K144G+L294I+L135V+FDH | +++++ | >99 | +++++ | >99 | +++++ | >99 | +++++ | >99 |

| | | | | | | | | |
|---|---|---|---|---|---|---|---|---|
| Note: - represents 0.5-20%, + represents 20-40%, ++ represents 40-60%, +++ represents 60-80%, ++++ represents 80-90%, and +++++ represents more than 90%. | | | | | | | | |

After the reaction was complete, the temperature of the system was reduced to 10-30 °C, 10 N of sodium hydroxide was used to adjust pH=9.8-10.2, boc anhydride was added, the temperature was kept at 10-30 °C for the reaction (pH=9.8-10.2). After the reaction, 35% sulfuric acid was used to adjust pH=7.5-8.5, 10 volumes of isopropyl acetate was added, and then 35% sulfuric acid was used to adjust pH=1.8-2.2. It was stirred for 5-10 min, then diatomite was added, the system passed through a pressure filter tank paved with the diatomite, and a filter cake was washed with 5 volumes of isopropyl acetate. After being combined, a filtrate was adjusted to pH=8.2-8.5 by using 10 N of sodium hydroxide. After being stirred for 1 h, solution was stilly placed for separation, 15 volumes of isopropyl acetate was added into a lower aqueous phase, 35% sulfuric acid was used to adjust pH=1.8-2.2, the temperature of the system was raised to about 40°C. After being stirred for 3 h at a constant temperature, solution was stilly placed for 1 h and separated, 15 V of isopropyl acetate was added into an aqueous phase, the temperature was adjusted to about 40°C and kept for 3 h, and then solution was separated after being stilly placed for 1 h. Organic phases were combined, the temperature was controlled at T≤ 50°C, and it was concentrated to about 9 volumes. 20 volumes of n-heptane was added at 82-89 °C within 10-15 min, the system was gradually cooled, the jacket temperature was controlled at 70 °C and kept for 1 h, the system was continued to cool to 40 °C. After the temperature was kept for 0.5 h, it was continued to cool to 0-5°C, the temperature was kept for 1 h. The system was suction-filtered, a filter cake was drip-washed with 10 v of n-heptane, and a product was dried at 55°C. 0.8 g of the product was obtained by weighing, and the yield was 80%.

In the present disclosure, other arbitrary combinations of the mutation sites may also have the better effects, and the mutation sites repeated on other amino acid dehydrogenase with the higher homology should also have the better effects.

The above are only preferred embodiments of the present disclosure, and are not intended to limit the present disclosure. For those skilled in the art, the present disclosure may have various modifications and changes. Any modifications, equivalent replacements, improvements and the like made within the spirit and principle of the present disclosure shall be included in the scope of protection of the present disclosure.

## Claims

1. An amino acid dehydrogenase mutant having an amino acid sequence obtained by the mutation of the amino acid sequence shown in SEQ ID NO: 1, and the mutation comprises a mutation site K144G.

2. The amino acid dehydrogenase mutant according to claim 1, wherein the mutation comprises any one of the following mutation site combinations: K144G+L294I, K144G+L294I+L135V, K144G+L294I+T115I,K144G+L41I,K144G+L41K,K144G+L41L,K144G+L41S,K144G+L41T, K144G+G42V, K144G+G43N,K144G+G114S,K144G+T115I,K144G+T115L,K144G+T115V. K144G+M117A, K144G+M117L, K144G+M117T, K144G+M117V, K144G+M117Y, K144G+G118A, K144G+G118H, K144G+G118N, K144G+G118Q, K144G+G118S, K144G+G118Y . K144G+T119A , K144G+T119C . K144G+T119E , K144G+T119L , K144G+T119N . K144G+T119S , K144G+P121M, K144G+P121S , K144G+P121T , K144G+P121W , K144G+L135I, K144G+L135V , K144G+K184H , K144G+K184M , K144G+K184N , K144G+K184S , K144G+G293A , K144G+G293P , K144G+G293S or K144G+L294V.

3. The amino acid dehydrogenase mutant according to claim 1, wherein the amino acid dehydrogenase mutant has an amino acid mutation of K144G + L294I + L135V, and is co-expressed with formate dehydrogenase FDH.

4. A DNA molecule encoding the amino acid dehydrogenase mutant according to any one of claims 1 to 3.

5. A recombinant plasmid containing the DNA molecule according to claim 4.

6. The recombinant plasmid according to claim 5, wherein the recombinant plasmid is pET-22b(+), pET-22b(+), pET-3a(+), pET-3d(+), pET-11a(+), pET-12a(+), pET-14b, pET-15b(+), pET-16b(+), pET-17b(+), pET-19b(+), pET-20b(+), pET-21a(+), pET-23a(+), pET-23b(+), pET-24a(+), pET-25b(+), pET-26b(+), pET-27b(+), pET-28a(+), pET-29a(+), pET-30a(+), pET-31b(+), pET-32a(+), pET-35b(+), pET-38b(+), pET-39b(+), pET-40b(+), pET-41a(+), pET-41b(+), pET-42a(+), pET-43a(+), pET-43b(+), pET-44a(+), pET-49b(+), pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-8, pUC-18, pRSFDuet1, or pUC-19.

7. A host cell comprising the recombinant plasmid according to claim 5 or 6.

8. The host cell according to claim 7, wherein the host cell comprises a prokaryotic cell or a eukaryotic cell.

9. The host cell according to claim 8, wherein the prokaryotic cell is *Escherichia coli* BL21 cell or *Escherichia coli* DH5α competent cell, and the eukaryotic cell is yeast.

10. A method for producing an amino acid, comprising a step of catalyzing reductive amination of a ketone compound by an amino acid dehydrogenase, wherein the amino acid dehydrogenase is the amino acid dehydrogenase mutant according to any one of claims 1 to 3.

11. The method according to claim 10, wherein the ketone compound is 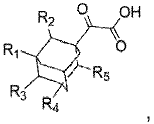 the reductive amination reactant is 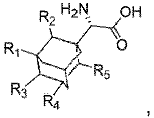 wherein R₁ represents -OH, -F, -Cl, -Br, or -CH₃, and R₂, R₃, R₄, R₅ each independently represents -H, -OH, -F, -Cl, -Br, or -CH₃.

12. The method according to claim 11, wherein the ketone compound is

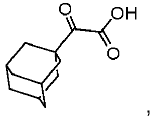

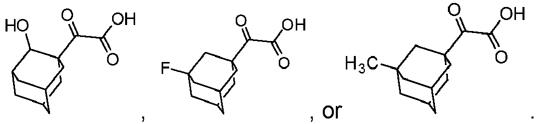

13. The method according to claim 11, wherein the amino donor is ammonium formate, ammonium chloride, ammonium carbamate, ammonium carbonate, ammonium bicarbonate, aqueous ammonia, ammonium oxalate, ammonium hydrogen oxalate, ammonium lactate, or ammonium phosphate.

14. The method according to claim 11, wherein the reductive amination catalyzing by the amino acid dehydrogenase has a condition of 30°C to 50°C, a stirring speed of 50 rpm to 250 rpm, and a concentration of formate dehydrogenase of 0.1 mg/mL to 1 mg/mL.
